# EUROPÄISCHE PATENTSCHRIFT

(11) **EP 1 804 963 B1**
(45) Veröffentlichungstag und Bekanntmachung des Hinweises auf die Patenterteilung: **09.09.2015**
(21) Anmeldenummer: 05798523.6
(22) Anmeldetag: 10.10.2005
(51) Int. Cl.: B01J 23/44, B01J 23/52, B01J 23/66, B01J 37/02, B01J 12/00, B01J 19/24, B01J 23/58, C07C 67/055, B01J 19/00

(54) **REAKTOR UND VERFAHREN ZUR SYNTHESE VON VINYLACETAT IN DER GASPHASE**
REACTOR AND METHOD FOR SYNTHESISING VINYL ACETATE IN THE GASEOUS PHASE
REACTEUR ET PROCEDE POUR LA SYNTHESE D'ACETATE DE VINYLE EN PHASE GAZEUSE

(30) Priorität: 15.10.2004 DE 102004050585
(43) Veröffentlichungstag der Anmeldung: 11.07.2007
(60) Teilanmeldung: 10004216.7
(73) Patentinhaber: ThyssenKrupp Industrial Solutions AG, 45143 Essen (DE); Evonik Degussa GmbH, 45128 Essen (DE)
(72) Erfinder: BÜKER, Karsten, 44139 Dortmund (DE); SCHIRRMEISTER, Steffen, 45472 Mülheim an der Ruhr (DE); LANGANKE, Bernd, 59439 Holzwickede (DE); MARKOWZ, Georg, 63755 Alzenau (DE); HAUSMANN, Ralf, 63607 Wächtersbach (DE); GEISSELMANN, Andreas, 63075 Offenbach (DE)
(74) Vertreter: Bülle, Jan
(86) Internationale Anmeldenummer: PCT/EP2005/010883
(87) Internationale Veröffentlichungsnummer: WO 2006/042659

(56) Entgegenhaltungen:
- EP-A- 1 008 385
- US-A- 5 854 171
- US-A- 6 013 834
- US-A1- 2002 028 164
- US-A1- 2002 031 455
- US-A1- 2003 180 216

## Beschreibung

Die Erfindung richtet sich auf einen Synthesereaktor zur Herstellung von monomerem Vinylacetat (VAM), in welchem gasförmiges Ethylen und gasförmige Essigsäure sowie Sauerstoff oder Sauerstoff enthaltende Gase katalytisch reagieren, wobei der Synthesereaktor ein Wandreaktor ist, in welchem die katalytische Synthese In einer Vielzahl von Reaktionsräumen erfolgt, die in Bezug auf den freien Strömungsquerschnitt mindestens in einer Dimension kleiner als 2000 µm, vorzugsweise kleiner als 1000 µm sind und deren indirekt gekühlten Wände mit einem Palladium-Gold Katalysator beschichtet sind.

Das Verfahren zur Synthese von Vinylacetat aus Ethylen ist im Stand der Technik umfassend beschrieben. Hierzu werden Ethylen, Essigsäure und molekularer Sauerstoff beziehungsweise O₂-enthaltende Gase, gegebenenfalls unter Zugabe von Inertgasen wie z.B. CO₂, bei Temperaturen von 100 °C bis 250 °C und erhöhtem Druck in Gegenwart eines Katalysators zur Reaktion gebracht wird. Dies geschieht, indem man das Prozessgas über eine Katalysatorschüttung leitet. Für diese stark exotherme Reaktion wird üblicherweise ein Katalysator eingesetzt, welcher Palladium, Gold und Alkalimetalle auf einem oxidischen Träger enthält. Der Katalysator liegt in Gestalt von Formkörpern wie z.B. Kugeln, Granulat, Tabletten oder Extrudaten vor, auf dem die katalytisch aktiven Substanzen in einer schalenförmigen Außenzone aufgetragen sind.

EP 1 106 247 B1 beschreibt ein solches Verfahren und einen geeigneten Katalysator, wobei der Trägerkatalysator einen idealen Pd-Anteil von 0,3 bis 4,0 Gew.-% und einen Au-Gehalt von 0,1 bis 2,0 Gew.-% aufweist. Die Katalysatordicke auf dem Träger wird mit maximal 1mm und bevorzugt kleiner als 0,5 mm angegeben. In EP 1 106 247 B1 werden Produktivitäten genannt (in der Schrift als Aktivität bezeichnet), welche maximal 225,5 g_{VAM}/kg_{Kat}*h betragen. Eine hohe Selektivität wird in der Schrift zwar genannt, aber nicht in Abhängigkeit von dem gewählten Trägerkatalysator oder dem Präparationsverfahren beschrieben oder quantifiziert. In EP 0 987 058 B1 wird ein Trägerkatalysator auf Basis von Siliziumdioxid beschrieben, welcher eine spezielle Trägergeometrie aufweist.

Verschiedene Katalysatoren, welche im Stand der Technik zur Synthese bekannt sind, werden in DE 190 20 390 genannt. In DE 198 34 569 A1 wird eine Dotierung mit Hafnium vorgeschlagen, welche zu hohen Produktivitäten von bis zu 1100 g_{VAM}/I_{Kat} * h bei 9 bar, 170° C und 0,5 Gew.-% Hf-Beladung geführt hat.

Die DE 199 14 066 offenbart einen Katalysator, in welchem Barium oder Cadmium als Dotierungselemente sowie unterschiedliche Metalloxide im Trägerkörper eingesetzt werden. Cadmium ist aus Umweltgesichtspunkten nachteilig.

Weiterhin ist aus DE 196 19 961, EP 0 916 402 A1 oder EP 0 997192 B1 bekannt, Trägerkörper hinsichtlich ihrer äußeren Form zu optimieren. Bekannt sind Presslinge, Zylinder, Ringe und weitere Formen, welche bei der VAM-Synthese zum Einsatz kommen. In EP 1 323 469 A3 wird ein Formkörper beschrieben, welcher als pyrogen hergestellter Siliziumdioxid-Körper spezielle offene Strukturen aufweist. In EP 1 106 247 B1 werden für derartige Formkörper äußere Abmessungen von 2-15 mm angegeben. Die Beladung wird mit 0,2-1.5 Gew.-% Au, 0,3-4,0 Gew.-% Pd und 3,5-10 Gew.-% Kaliumacetat angegeben.

In EP 0 997 291 B1 werden Trägermaterialien genannt, welche aus mindestens zwei Komponenten der Gruppe SiO₂, Al₂O₃, TiO₂ und ZrO₂ bestehen. Gemäß der vorgenannten Schrift können diese Trägermaterialien in einer beliebigen Form beispielsweise als Zylinder, Kugeln oder Ringe vorliegen, welche als Strangpresslinge, Extrudate oder Tabletten hergestellt werden. Auch in der EP 0 723 810 B1 wird ein Trägermaterial offenbart, welches die Elemente Zirkon und Titan enthält. Als Vorteil der Erfindung wird in den Beispielen aufgezeigt, dass eine Produktivität von 225 g_{VAM}/kg_{Kat} * h erreicht wird.

Es ist weiterhin im Stand der Technik bekannt und beschrieben, dass eine Reihe von Nebenprodukten bei der VAM-Synthese gebildet-werden. In DE 199 20 390 werden hier die Stoffe CO₂, Ethylacetat und Hochsieder, wie z.B. Ethylidendiacetat, Ethylenglykol und dessen Acetate oder Diacetoxyethylen genannt. Es wird in der vorgenannten Schrift ein Katalysator beschrieben, welcher durch die Hinzunahme von Vanadium die Selektivität hinsichtlich der Hochsieder vorteilhaft beeinflusst.

Weiterhin ist bekannt, dass bei der Produktion von Vinylacetat erhebliche Anstrengungen erforderlich sind, das Produkt im Anschluss an den Syntheseschritt von den Edukten und Nebenprodukten zu trennen. Die Abtrennung geschieht üblicherweise mittels einer der Synthese nachgeschalteten Destillation und weiterer Schritte zur Trennung der Stoffströme. In DE 39 34 614 A1 wird eine Vinylacetatsynthese mit unterschiedlichen Prozessschritten zur Produktnachbehandlung beschrieben, wobei als bekannte Grenzwerte von Ethylacetat 1000-2000 Gew.-ppm angegeben werden und ein zu erreichender Restgehalt an Ethylacetat von 150 Gew.-ppm im Produkt genannt wird.

In EP 0 072 484 wird ein Verfahren offenbart, welches durch Zugabe von geringen H₂O-Mengen in einen Vinylacetat-Rücklaufstrom und dessen Einleitung in die Destillation zu einer verbesserten Entwässerung führt. In DE 198 25 254 wird ein analoges Reinigungsverfahren beschrieben, bei welchem neben Wasser auch Essigsäure in diesen Rücklaufstrom gegeben wird, wodurch eine Verringerung des Ethylacetatgehaltes erreicht wird. Diese Verfahren sind der Synthese nachgeschaltete Prozessschritte und verringern die Bildung von Nebenprodukten durch zusätzliche Maßnahmen.

In US 2002/0031455 A1 wird ein Reaktor für endotherme Reaktionen offenbart, der aus einer monolithischen Keramik besteht und in paralleler Anordnung Reaktions- und Heizkanäle aufweist, wobei die Reaktionskanäle mit einem katalytischen Material beschichtet sein können.

In US 2003/0180216 A1 wird ein Mikroreaktor offenbart, der eine Reaktionskammer aufweist, die mit einem Wärmetauscherkanal in thermischen Kontakt steht. Die Reaktionskammer kann einen Katalysator aufweisen.

In US 6,013,834 wird ein konventioneller Festbettreaktor zur Herstellung von Vinylacetat offenbart, in dem ein Synthesegas durch eine Vielzahl von mit einem Pd/Au und Alkalimetallenthaltenen Schalenstrukturkatalysator gefüllten Rohren strömt und zum Vinylacetat reagiert.

Nach wie vor besteht ein Mangel an einem Verfahren, das sowohl eine hohe Produktivität als auch eine hohe Selektivität des Katalysators ermöglicht. Hinsichtlich der Selektivität bedarf es insbesondere solcher Verfahren, die die Bildung von aufwändig abzutrennenden Nebenprodukten wie Ethylacetat unterdrücken.

Aufgabe der Erfindung ist es somit, den Mangel im Stand der Technik zu beheben und die Bildung von Nebenprodukten zu verringern und zeitgleich eine hohe Selektivität und Produktivität zu erreichen. Die Produktivität ist dabei bestimmt als die gebildete Masse an Vinylacetat pro Katalysatormasse und Zeiteinheit mit der Einheit g_{VAM}/kg_{Kat}*h, wobei die Masse des Katalysators ohne Binder berechnet wurde. Die erfindungsgemäße Vorrichtung und das erfindungsgemäße Verfahren lösen diese Aufgabenstellung im Stand der Technik dadurch, dass ein Synthesereaktor zum Einsatz zur Herstellung von Vinylacetat eingesetzt wird, in welchem gasförmiges Ethylen und Essigsäure sowie Sauerstoff oder Sauerstoff enthaltende Gase katalytisch reagieren, wobei als Synthesereaktor ein Wandreaktor eingesetzt wird. Dieser Wandreaktor weist eine Vielzahl von Reaktionsräumen auf, in welchen die katalytische Reaktion erfolgt. Mindestens eine Wand in jedem dieser Reaktionsräume ist mit einem Katalysator beschichtet und indirekt gekühlt. Der Katalysator, der 0,3 bis 10 Gew,-% Palladium, 0,2 bis 5 Gew.-% Gold und 0,4 bis 6 Gew.-% einer Alkalimetallverbindung auf einem oxidlschen Trägermaterial enthält, ist mittels eines Binders auf die Wand der Reaktionsräume aufgebracht. Die Dimensionierung der Reaktionsräume ist derart gewählt, dass der freie Strömungsquerschnitt in jedem dieser Reaktionsräume mindestens in einer Dimension kleiner als 2000 µm ist.

In einer vorteilhaften Ausgestaltung der Vorrichtung bestehen die Reaktionsräume aus einer Vielzahl von Rohren oder aus gestapelten Platten, welche eine Vielzahl von Spalten aufweisen, wobei die Rohre oder Spalten zueinander eine beliebige Ausrichtung aufweisen können und idealerweise parallel zueinander verlaufen.

Dabei ist es von Vorteil, wenn in einer optimierten Ausgestaltung nur genau eine Dimension des Reaktionsraumes kleiner 1000 µm ist. Dies ist bei rohrförmigen Reaktionsräumen der freie Strömungsdurchmesser und bei spaltförmigen Reaktionsräumen entweder die Spalthöhe oder die Spaltbreite des freien Strömungsquerschnitts. Die sonstigen Dimensionen nicht in diesen sehr kleinen Dimensionen auszuführen hat den Vorteil, dass Dichtheit, mechanische Belastungen und Fertigungsvorgänge leichter beherrscht werden können.

Der Palladium-Gehalt das erfindungsgemäßen Katalysators beträgt vorzugsweise 0,8 bis 5 Gew.-% während der Gold-Gehalt vorzugsweise 0,4 bis 2,5 Gew.-% beträgt. Hierin liegt ein wesentlicher Vorteil der Erfindung, dass nämlich sehr viel höhere Pd- und Au-Gehalte und damit Produktivitäten realisiert werden können, als dies in den aus dem Stand der Technik bekannten Formkörper-Katalysatoren der Fall ist. Dies hat seine Ursache zum einen darin, dass aufgrund der Anhaftung des Katalysators an der Reaktorwand und der indirekten Wandkühlung durch den metallischen Träger, ein sehr vorteilhafter Wärmeabtransport realisiert wurde.

Zum anderen können die Metalle in der gesamten Katalysatorschicht verteilt werden, da durch die geringe Schichtdicke und die hohe Porosität des Trägermaterials keine Massentransportwiderstände bei der Reaktion vorliegen, die negativ hinsichtlich der Aktivität wirken. Eine Schalenstruktur, wie sie bei Formkörpern üblich ist, liegt nicht vor. Die örtliche Edelmetallkonzentration in der Katalysatorschicht ist bedingt durch diese weitgehend homogene Verteilung lokal nicht überhöht, so dass die katalytisch aktive Metalloberfläche als Solche optimal ist und entsprechend hohe Aktivitäten erreicht werden.

Weiterhin weist der Katalysator vorzugsweise einen Alkalimetall-Gehalt von 1 bis 4 Gew.-% des Alkalimetalls auf. Als Alkalimetall wird vorrangig Kalium eingesetzt, welches bevorzugt in der Form seines Acetates vorliegt.

In einer weiteren vorteilhaften Ausgestaltung der erfindungsgemäßen Vorrichtung enthält der eingesetzte Katalysator eines oder mehrere Elemente aus der Gruppe der Erdalkalimetalle, Lanthanoiden, Vanadium, Eisen, Mangan, Kobalt, Nickel, Kupfer, Cer, Platin, wobei der Gesamtanteil dieser Elemente 3 Gew.-% nicht übersteigt.

Vorzusgweise weist das oxidische Trägermaterial als Hauptkomponente ein Oxid aus der Gruppe SiO₂, Al₂O₃, TiO₂ und ZrO₂ auf. In einer vorteilhaften Ausgestaltung enthält das Trägermaterial weitere Oxide als Nebenkomponenten. Als natürliche Mlschoxide können belspielsweise Bentonite eingesetzt werden.

Eine vorteilhafte Ausführungsvariante der erfindungsgemäßen Vorrichtung besteht darin, dass das Basismaterial der Reaktionsräume mindestens teilweise aus Edelstahl besteht, und der Katalysator unter Verwendung eines oxidischen oder organischen Binders auf die zu beschichtenden Edelstahlwände aufgetragen wird. Hierbei können Binder aus den Gruppen der Metalloxid-Sole, Cellulosederivate oder Alkallmetall-Silikate, wie beispielsweise Siliziumoxid-Sole, Methylcellulosen oder Wasserglas eingesetzt werden.

Von der Erfindung ist ebenfalls ein Verfahren unter Einsatz des vorbeschriebenen Reaktors umfasst, wobei der Reaktor nahezu isotherm betrieben wird, und der maximale Temperaturanstieg zwischen Eintritt und Austritt des Synthesereaktors 5 K, vorzugsweise 2 K beträgt. Dieser geringe Temperaturanstieg ist nicht nur in Bezug auf die Differenz der Ein- und Austrittstemperaturen gegeben, sondern gilt für alle Bereiche innerhalb des Reaktors gleichermaßen. Bei den im Stand der Technik verwendeten Rohrreaktoren mit Katalysatorschüttungen, bilden sich in den einzelnen Reaktionsrohren starke radiale Wärmegradienten aus, welche bei dem erfindungsgemäßen Reaktor nicht auftreten können, da der Katalysator als Wandbeschichtung vorliegt und durch die intensive indirekte Wandkühlung ein vollflächiger, optimaler Wärmeabtransport sichergestellt ist.

Das erfindungsgemäße Verfahren zeichnet sich weiterhin dadurch aus, dass in den Reaktionsräumen eine Temperatur von 100 bis 250 °C und vorzugsweise von 150 bis 200 °C vorliegt, wobei der Druck im Bereich von 1 bis 12 bar liegt und vorzugsweise 6 bis 10 bar absolut beträgt.

Vorteilhafter Welse kann das erfindungsgemäße Verfahren bei explosiven Prozesszuständen durchgeführt werden, und es müssen hinsichtlich der optimalen Betriebsparameter keine Einschränkungen hingenommen werden, welche daher resultieren, dass explosive Zustände erreicht werden. Als explosive Zustände werden Gasmischungen verstanden mit einer Zusammensetzung, die unter den im Prozess vorliegenden Bedingungen wie Druck und Temperatur in einem definierten Volumen zur Explosion gebracht werden können. Von besonderer technischer Relevanz ist die untere Explosionsgrenze für Sauerstoff, die in US 3,855,280 mit typischerweise 8 % quantifiziert wird. Mit dem erfindungsgemäßen Verfahren sind Prozesszustände umfasst, bei welchen ein O₂-Gehalt über 7 Vol.-% im Prozessgas vorliegt.

Eine vorteilhafte Weiterentwicklung besteht darin, dass die Katalysatorschicht mittels eines oxidischen oder organischen Binders auf die Wände der Reaktionsräume aufgetragen wird, wobei der Anteil des Binders in der Katalysatorschicht ≤ 50 Gew.-% beträgt und bevorzugt Binder aus den Gruppen Metalloxid-Sole. Cellulosederivate oder Alkalimetall-Silikate wie beispielsweise Siliziumoxid-Sole, Methylcellulosen oder Wasserglas eingesetzt werden.

Eine optimierte Ausgestaltung des erfindungsgemäßen Katalysators besteht,darin, dass alle vorgenannten Dotierungs- und Aktivierungselemente homogen im gesamten Volumen der Katalysatorschicht verteilt sind und keine Schichtung vorliegt, wie dies bei den aus dem Stand der Technik bekannten Schalenkatalysatoren üblich ist.

Zur Untersuchung der erfindungsgemäßen Vorrichtung wurde der Katalysator analog der Präparation in EP 1 008 385 A1 hergestellt, wobei ein pulverförmiger SiO₂-haltiger Träger eingesetzt wurde, der in der Zusammensetzung mit einem üblichen Schüttgutkatalysatorträger identisch ist. Anschließend wurde der Katalysator auf zwei Edelstahlplatten mit Stegen appliziert und mit Kaliumacetat aktiviert. Die Plattenpaare werden so miteinander verbunden, dass die Katalysatorschichten einander gegenüberliegen und ein Spalt von ca. 500 µm entsteht. Dieses Plattenpaar wird in einen druckfesten Reaktor eingebracht, welcher mit Öl beheizt und bei 155 °C nahezu vollständig isotherm betrieben wurde. Der Temperaturanstieg war kleiner als 1 K, wobei an 5 Messpunkten entlang des Strömungsverlaufes gemessen wurde.

Der Druck im Reaktor lag im Bereich von 5 bar bis 9 bar absolut. Ethylen, Sauerstoff, Methan und Helium wurden gasförmig dosiert, wobei das Methan als interner Standard für die Analytik diente. Die Essigsäure wurde flüssig dosiert und vor dem Reaktor verdampft. Das in den Reaktor eintretende Gasgemisch hatte folgende Zusammensetzung, angeben In Volumenprozent: 63,2 % Ethylen, 5,7 % Sauerstoff, 4,2 % Methan, 9,6 % Helium und 17,2 % Essigsäure. Bei Start der Testung wird zunächst sauerstofffrei gefahren, der Sauerstoffgehalt kann jedoch schnell im Verlauf einer Stunde auf den Standardgehalt angehoben werden. Die Analytik erfolgt über Gaschromatographie und einen CO₂-Detektor.

In einem ersten Versuch, dessen Ergebnisse in Tabelle 1 dargestellt sind, wurde die Abhängigkeit der Produktivität vom Palladium- und Goldgehalt des Katalysators untersucht. Die Messungen wurden bei 5 bar und bei 9 bar sowie bei zwei unterschiedlichen Gaslasten im Katalysatorbett durchgeführt. Die Gaslast wurde gemessen als Produktgasvolumen pro Katalysatormasse und Zeit mit der Einheit I_{VAM}/kg_{Kat}*h, wobei nur die aktive Katalysatormasse betrachtet wurde, also ohne den Binderanteil. Dabei wurde ein nach vorstehend genannter Methode präparierter Katalysator eingesetzt.

**Tabelle 1: Einfluss des Edelmetallgehaltes auf die Produktivität**

| Pd/Au-Gehalt in Masse-% | Produktivität [g_{VAM}/kg_{Kat}*h] | Produktivität g_{VAM}/kg_{Kat}*h] |
|---|---|---|
| | bei p = 5 bar und | Bei p = 9 bar und |
| | Gaslast = 12.000 l/kg*h | Gaslast = 24.000 l/kg*h |
| 0,83 / 0,36 | 450 | 750 |
| 2,50 / 1,10 | 830 | 1300 |
| 5,00 / 2,20 | 1120 | 2100 |

Es kann der Tabelle 1 entnommen werden, dass die Produktivität mit steigendem Pd-Au-Gehalt ansteigt. Hohe Werte sind zwar zum Teil im Stand der Technik als im Prinzip vorteilhaft berichtet, allerdings ist dem Fehlen an entsprechenden Versuchen oder Beispielen zu entnehmen, dass in dem konventionellen Verfahren die Temperaturen mit einem derart aktiven Katalysator nicht beherrscht werden konnten. Hierin ist ein Vorteil der Erfindung zu erkennen, dass nämlich die Temperaturentwicklung aufgrund einer hohen Pd-Au-Beladung nicht mehr limitierend für das Verfahren ist.

In Versuch 2 wurde das Verhalten des erflndungsgemäßen Reaktors bei unterschiedlichen Gesbalastungen im Katalysatorbett untersucht.

**Tabelle 2: Einfluss der Gaslast eines Pd/Au-Wandkatalysators (0,83% Pd / 0,36% Au) auf die Produktivität**

| Gaslast in l/Kg*h bei 5 bar | Produktivität in g_{VAM}/kg_{Kat}*h |
|---|---|
| 3000 | 300 |
| 6000 | 350 |
| 12000 | 400 - 450¹⁾ |
| 24000 | 450 - 480¹⁾ |

| | |
|---|---|
| ¹⁾ Messwert s. auch Tabelle 3 | |

Es ist aus Tabelle 2 zu entnehmen, dass ein Vorteil der Erfindung darin besteht, das Verfahren bei einer Gaslast bis ungefähr 12.000 l/kg*h zu betrieben. Dabei ist der Druckverlust im erfindungsgemäßen Reaktor aufgrund der umlenkungs- und störungsarmen Strömungsführung deutlich geringer, als in dem gekannten Rohrreaktor, welcher eine Katalysatorschüttung aufweist. Eine weitere Erhöhung der Gaslast über 12.000 l/kg*h bringt keine wesentliche Steigerung der Produktivität.

Gemeinsam mit den Erkenntnissen aus dem Versuch 3, in welchem steigende Pd-Au-Gehalte, steigende Drücke und steigende Gaslasten realisiert wurden, ist zu erkennen, dass alle diese drei Parameter einen positiven Einfluss auf die Produktivität haben und deren Variation vom erfindungsgemäßen Reaktor oder dem Verfahren nicht limitiert werden.

**Tabelle 3 Einfluss des Betriebsdruckes und der Gaslast bel unterschiedlichen Pd-Gehalten auf die Produktivität**

| Pd/Au-Gehalt in Masse-% | Produktivität [g_{VAM}/kg_{Kat}*h] | Produktivität [g_{VAM}/kg_{Kat}*h] | Produktivität [g_{VAM}/kg_{Kat}*h] |
|---|---|---|---|
| | Druck 5 bar | Druck 5 bar | Druck 9 bar |
| | Gaslast = 12.000 l/kg*h | Gaslast = 24.000 l/kg*h | Gaslast = 24.000 l/kg*h |
| 0,83/0,36 | 450 | 480 | 760 |
| 2,50/1,10 | 830 | 850 | 1350 |
| 5,00/2,20 | 1120 | nicht gemessen | 2100 |

Im Versuch 4 wurde die Temperaturabhängigkeit des Verfahrens untersucht, wobei die Selektivität für Vinylacetat bezogen auf Ethylen und die Produktivität an Vinylacetat betrachtet wurde. Es wurde dabei gefunden, dass hinsichtlich der Selektivität ein Optimum durchlaufen wird, was beispielhaft bei einem Druck von 9 bar und der gewählten Katalysatorbeladung von 0,83 Gew.-% Pd und 0,36 Gew.-% Au im Bereich von 160°C bis 170°C liegt, wobei Selektivitäten von fast 98 % erreichbar sind. Die Produktivität ist mit weiter steigender Temperatur ebenfalls zu steigern, so dass eine Produktivität bis zu 1.400g_{VAM}/kg_{Kat}*h bei 185°C bei dieser niedrigen Pd-Au-Beladung erreicht wurde.

**Tabelle 4 Einfluss der Betriebstemperatur bei p = 9 bar und Gaslast = 24.000 l/kg*h (Wandkatalysator mit 0,83% Pd /0,36% Au)**

| | T = 155°C | T =165°C | T = 175°C | T = 185°C |
|---|---|---|---|---|
| Produktivität VAM g_{VAM}/kg_{Kat}*h | 700 | 900 | 1150 | 1400 |
| Selektivität VAM in % (bezogen auf C₂H₄) | 97,4 | 97,4 | 96,8 | 95,6 |

In Versuch 5 wurde versucht, das erfindungsgemäße Verfahren unter explosiven Gaszuständen durch Erhöhung der Sauerstoffkonzentration zu betreiben. Die Testbedingungen 3 und 4 liegen im explosiven Bereich hinsichtlich der O₂-C₂H₄-Verhältnisse. Es ist der Vorteil der Erfindung darin zu sehen, dass zur Steigerung der Produktivität explosive Prozesszustände zur Optimierung des Verfahrens ganz gezielt eingestellt werden können.

**Tabelle 5 Auswirkung der Erhöhung der O₂-Konzentration auf die Produktivität an VAM bei p = 5 bar und Gaslast = 12.000 l/kg*h (Wandkatalysator mit 0,83% Pd / 0,36% Au)**

| | Testbedingung 1 | Testbedingung 2 | Testbedingung 3 | Testbedingung 4 |
|---|---|---|---|---|
| | | | | |
| Vol.-% C₂H₄ | 63,2 | 61,2 | 59,5 | 57,8 |
| Vol.-% 0₂ | 6,0 | 8,7 | 11,3 | 13,8 |
| Vol.-% HOAc | 17,2 | 16,7 | 16,2 | 15,7 |
| Inerte (He, CH₄) | 13,9 | 13,5 | 13,1 | 12,7 |
| Raumzeitausbeute VAM g_{VAM}/kg_{Kat}*h | 430 | 630 | 700 | 1100 |

In Versuch 6 wurde der Einfluss der Schichtdicke auf die Produktivität und die Flächen-Zelt-Ausbeute an Vinylacetat untersucht. Die nahezu konstante massenspezifische Aktivität des Katalysators bei erhöhter Schichtdicke zeigt, dass Massentransportwiderstände innerhalb der Schicht nur eine untergeordnete Rolle spielen. Ein weiterer Vorteil der Erfindung ist also, dass keine schalenförmige Verteilung des Edelmetalls gewählt werden muss, die mit hohen lokalen Edelmetallkonzentrationen und damit niedrigen Edelmetalloberflächen einhergeht.

**Tabelle 6 Elnfluss der Schichtdicke des Wandkatalysators mit 2,5 Masse % Pd auf die Flächenzeitausbeute an VAM**

| Schichtdicke in µm | Produktivität VAM g_{VAM}/kg_{Kat}*h | Flächenzeitausbeute VAM g_{VAM}/m²_{Kat} |
|---|---|---|
| 300 | 850 | 130 |
| 500 | 780 | 196 |

Es konnte bei höheren Beladungen des Wandkatalysators eine Produktivität an VAM bei Drücken bis 9 bar von bis zu 5 kg_{VAM}/kg_{Kat}*h beobachtet werden.

In einer weiteren Versuchskampagne wurde ein Pd-Au-Katalysator verwendet, welcher in Anlehnung an das in EP 0 723 810 A1 beschriebene Verfahren präpariert wurde. Im Gegensatz zu den in der EP 0 723 810 A1 beschriebenen Formkörpern wurden für die nachstehend beschriebenen Versuche Partikel mit einer Korngröße von 50-150 µm verwendet. Das Basismaterial ist dabei mit dem der Formkörper identisch. Es wurde weiterhin die Ethylenkonzentration im Eduktstrom gegenüber den Versuchen 1 bis 6 vermindert und durch einen entsprechenden Volumenanteil an Inertgas und einen geringen Anteil an Wasserdampf ersetzt. Eine Änderung des Ethylenanteils im Eduktstrom in dem erfolgten Umfang hat einen zu vernachlässigenden Einfluss auf die Synthesereaktion. Im Versuch 7 wurde der nach vorgenannter Methode präparierter Wandkatalysator verwendet, welcher 2,5 Gew.-% Pd und 1,1 Gew.-% Au enthielt. Als weitere Testbedingung wurde eine Gastemperatur von 155°C und ein Druck von 9 bar eingestellt. Die Zusammensetzung des Eduktgases wurde wie folgt gewählt:

| | |
|---|---|
| 49,2 Vol.% | Ethylen (C₂H₄) |
| 18,0 Vol.% | Essigsäure (HOAc) |
| 1,3 Vol.% | Wasser (H₂O) |
| 31,5 Vol.% | Sauerstoff + Inertgas (Helium + Methan) |

Die Messergebnisse sind in Tabelle 7 zusammengestellt.

**Tabelle 7: Einfluss der O₂-Konzentration auf die Produktivität und Selektivität**

| O₂ (%) | Gaslast | Produktivität | Selektivität VAM (bezogen auf C₂H₄) | C₂H₄-Umsatz |
|---|---|---|---|---|
| Vol% | l/kg_{Kat}*h | g_{VAM} / kg_{Kat}*h) | % | % |
| | | | | |
| 6,5 | 6000 | 1600 | 94,3 | 15,0 |
| 9,0 | 6000 | 1900 | 94.7 | 17,6 |
| 11,5 | 6000 | 2320 | 93,6 | 21,5 |
| 14,0 | 6000 | 2620 | 93,4 | 24,8 |
| 14,0 | 12000 | 3180 | 96,0 | 14,2 |

Versuch 7 zeigt, dass sich bei einer Raumgeschwindigkeit von 6.000 l/kg_{Kat}*h durch Erhöhung der Sauerstoff-Konzentration von 6,5 Vol.%, welche im nicht explosiven Bereich liegt, auf 14,0 Vol.% im Explosionsbereich, der Umsatz an Ethylen von 15% auf 24,8% erhöht. Dabei ist wirklich überraschend, dass diese deutliche Umsatzerhöhung nur zu einem sehr geringen Abfall der Selektivität von 94,3 auf 93,6% führt. Eigentlich ist bei der sehr hohen O₂-Konzentration im explosiven Bereich eine deutlich stärkere CO₂-Bildung aufgrund der parallelen Reaktion von Ethylen mit O₂ eben hierzu zu erwarten, welche sich aber überraschenderweise nicht einstellt, Insgesamt kommt es zu einer Steigerung der Produktivität von 1600 g_{VAM}/kg_{Kat}*h auf 2620 g_{VAM}/kg_{Kat}*h.

Bei einer Erhöhung der Raumgeschwindigkeit von 6.000 auf 12.000 l/kg_{Kat}*h kann die Produktivität noch weiter auf 3.180 g_{vAM}/kg_{Kat}*h gesteigert werden und die Selektivität bezüglich Ethylen erhöht sich gegenüber der Fahrweise bei 6.000 l/kg_{Kat}*h dabei sogar noch von 93,4 auf 96 %.

Im Versuch 8 wurde das aus dem Stand der Technik bekannte Verfahren unter Einsatz eines Schüttgutkatalysators im Rohrreaktor im Vergleich mit dem erfindungsgemäßen Verfahren unter Einsatz eines Mikroreaktors untersucht. Wie in Versuch 7 wurde der nach vorgenannter Methode präparierter Wandkatalysator verwendet, welcher ebenfalls 1,1 Gew.-% Au aufwies. Die Gastemperatur betrug 155°C und der Druck 9 bar, wobei eine Gaslast von 12.000 l/kg_{Kat}*h gewählt wurde.

Die Zusammensetzung des Eduktgases wurde wie folgt gewählt:

| | |
|---|---|
| 49,2 Vol.% | Ethylen (C₂H₄) |
| 18,0 Vol.% | Essigsäure (HOAc) |
| 1,3 Vol.% | Wasser (H₂O) |
| 6,5 Vol.% | Sauerstoff |
| 25,0 Vol.% | Inertgas (Helium + Methan) |

**Tabelle 8: Vergleich zwischen Wandkatalysator und dem Schüttgutkatalysator bei jeweils üblicher Pd-Beladung der unterschiedlichen Katalysatoren**

| | Produktivität | Selektvität VAM (bezogen auf C₂H₄) | C₂H₄-Umsatz |
|---|---|---|---|
| | g_{VAM}/kg_{Kat}*h | [%] | [%] |
| Wandkatalysator mit 2,5 Gew.-% Pd | 2.050 | 96,3 | 9,6 |
| Schüttgutkatalysator mit 0,56 Gew.-% Pd | 720 | 94,1 | 3.2 |

Der Wandkatalysator zeigt bei erhöhtem C₂H₄-Umsatz überraschenderweise auch eine höhere VAM-Selektivität bezüglich Ethylen als der Schüttgutkatalysator. Insgesamt führt das zu einer Produktivität des Wandkatalysators von 2050 g_{VAM}/kg_{Kat}*h im Vergleich zu 720 g_{VAM}/kg_{Kat}*hdes Schüttgutkatalysators. Für den Wandkatalysator konnte eine Metallbeladung gewählt werden, welche auf dem Schüttgutkatalysator nicht realisierbar ist, da eine derart hohe Beladung mit Pd und Au zu so starken lokalen Überhitzungen führt, so genannten "hot spots".

Bei dem Versuch 8 wurde weiterhin das Nebenproduktsprektrum des erfindungsgemäßen und des bekannten Verfahrens untersucht.

**Tabelle 9: Vergleich der Nebenproduktspektren; T=155 °C, p=9 bar, Gaslast =12.000 l/kg_{Kat}* h**

| Nebenprodukt | Wandkatalysator mg_{Nebenprodukt}/kg_{VAM} | Schüttgutkatalysator mg_{Nebenprodukt}/kg_{VAM} |
|---|---|---|
| | [ppm] | [ppm] |
| Acetaldehyd | 5.774 | 8.917 |
| Methytacetat | 137 | 1076 |
| Ethylacetat | 1931 | 4283 |
| 1.2-Ethandiolmonoacetat | 907 | 7819 |
| 1.1-Ethandioldiacetat / Ethyli-dendiacetat | 868 | 1.691 |
| 1.1-Ethendioldiacetat / Vinyli-dendiacetat | 2.038 | 7.731 |
| Summe 1.2-Diacetate | 2.906 | 9.422 |

Der Vergleich des Wandkatalysators mit dem Schüttgutkatalysator zeigt überraschenderweise, dass die Nebenproduktbildung im Fall des Wandkatalysators deutlich geringer ist als im Falle des Schüttgutkatalysators. Dies stellt einen wichtigen wirtschaftlichen Vorteil dar, da der Aufwand für die Reinigung und Trennung von Produkt und Nebenprodukten bei einer industriellen Anwendung beim Einsatz der erfindungsgemäßen Vorrichtung deutlich vermindert wird.

In einem weiteren Versuch 9 wurde die Standzeit des Katalysators anhand des Abfalls der Produktivität gemessen. Dabei waren die Testbedingungen hinsichtlich der Zusammensetzung des Eduktes und der Temperatur identisch mit Versuch 8, nur der Druck betrug für Versuch 9 5 bar und die Gaslast war für den Wandkatalysator und die Rohrreaktor unterschiedlich gewählt:

| | | |
|---|---|---|
| Reaktor mit Wandkatalysator | 2,5% Pd | Gaslast = 12.000 l/kg_{Kat}*h |
| Rohrreaktor mit Schüttgutkatalysator | 0,56% Pd | Gaslast = 6.000 l/kg_{Kat}*h |

**Tabelle 10: Vergleich der Standzeiten von Wand- und Schüttgutkatalysator**

| | Wandkatalysator | | Schüttgutkatalysator | |
|---|---|---|---|---|
| Standzeit | Produktivität | | Produktivität | |
| [h] | [g_{VAM}/kg_{Kat}*h] | Relative Produktivität (10h=100%) | [g_{VAM}/kg_{Kat}*h] | relative Produktivität (10h = 100%) |
| 10 | 2100 | 100 | 390 | 100 |
| 20 | 2150 | 102 | 370 | 95 |
| 100 | 2000 | 95 | 270 | 69 |

Der jeweilige Wert für die Produktivität nach 10 Stunden wurde als Basiswert genommen und die relative Veränderung zu diesem Basiswert in der Tabelle 10 dargestellt. Die Messdaten zeigen das überraschende Ergebnis, dass der Wandkatalysator während einer Dauer von 100 h im Vergleich zum Schüttgutkatalysator trotz deutlich höherer Produktivität kaum deaktiviert. Dies ist für die technische Anwendung ein erheblicher Vorteil des Wandkatalysators gegenüber dem bekannten Schüttgutkatalysator.

Überraschender Weise wurde festgestellt, dass eine Selektivität bezüglich Ethylen bis ca. 98 % erreicht werden konnte, bei gleichzeitig sehr hohen Umsätzen. Ergänzend hierzu wurde erstaunlicher Weise beobachtet, dass eine deutlich verminderte Menge an Nebenprodukten gebildet wird. Die hohe Vinylacetat-Selektivität und die geringe Bildungsrate der Nebenprodukte führt in der Folge zu einem erheblich verringerten Aufreinigungsbedarf des Produktes in dem erfindungsgemäßen Verfahren, verglichen mit den bekannten Verfahren im Stand der Technik und somit zu erheblichen wirtschaftlichen Vorteilen.

## Patentansprüche

1. Synthesereaktor zur Herstellung von Vinylacetat, in welchem gasförmiges Ethylen und Essigsäure sowie Sauerstoff oder Sauerstoff enthaltende Gase katalytisch reagieren,
- der Synthesereaktor ein Wandreaktor ist und
- eine Vielzahl von Reaktionsräumen aufwelst, in welchen die katalytische Reaktion erfolgt, wobei
- der freie Strömungsquerschnltt in jedem dieser Reaktionsräume mindestens in einer Dimension kleiner als 2000 µm ist, wobei
- mindestens eine Wand der Reaktionsräume mit einem Katalysator beschichtet ist, und
- mindestens eine Wand der Reaktionsräume indirekt gekühlt ist,
- der Katalysator, welcher 0,3 bis 10 Gew.-% Palladium, 0,2 bis 5 Gew.-% Gold und 0,4 bis 6 Gew.-% einer Alkalimetallverbindung auf einem oxidischen Trägermaterial enthält, mittels eines Binders haftend auf die Wand der Reaktionsräume aufgebracht ist.

2. Synthesereaktor nach Anspruch 1, **dadurch gekennzeichnet, dass** genau eine Dimension der Reaktionsräume kleiner als 1000 µm ist.

3. Synthesereaktor nach einem der Ansprüche 1 oder 2, **dadurch gekennzeichnet, dass** die Reaktionsräume aus einer Vielzahl von Rohren oder aus Platten angeordneten Spalten gebildet werden, wobei diese in beliebiger Ausrichtung angeordnet sein können und idealerweise parallel zueinander verlaufen.

4. Synthesereaktor nach einem der Ansprüche 1 bis 3, **dadurch gekennzeichnet, dass**
der Palladium-Gehalt des Katalysators 0,8 bis 5 Gew.-% beträgt.

5. Synthesereaktor nach einem der Ansprüche 1 bis 4, **dadurch gekennzeichnet, dass**
der Gold-Gehalt des Katalysators 0,4 bis 2,5 Gew.-% beträgt.

6. Synthesereaktor nach einem der Ansprüche 1 bis 5, **dadurch gekennzeichnet, dass** die Alkalimetaliverbindung einen Alkalimetall-Gehalt von 1 bis 4 Gew.-% aufweist.

7. Synthesereaktor nach einem der Ansprüche 1 bis 6, **dadurch gekennzeichnet, dass**
der Katalysator eines oder mehrere Elemente aus der Gruppe der Erdalkalimetalle, Lanthanoiden, Vanadium, Eisen, Mangan, Kobalt, Nickel, Kupfer, Cer, Platin enthält, wobei der Gesamtanteil dieser Elemente 3 Gew.-% nicht übersteigt.

8. Synthesereaktor nach einem der Ansprüche 1 bis 7, **dadurch gekennzeichnet, dass**
das oxidische Trägermaterial ein Metalloxid aus der Gruppe SiO₂, Al₂O₃, TiO₂ und ZrO₂ als Hauptkomponente ist, wobei in einer vorteilhaften Ausgestaltung das Trägermaterial weitere Oxide als Nebenkomponenten enthält, und das Trägermaterial ein natürliches Mischoxid aus der Gruppe der Bentonite sein kann.

9. Synthesereaktor nach einem der Ansprüche 1 bis 8, **dadurch gekennzeichnet, dass**
der Binder ein oxidischer oder ein organischer Binder ist, wobei bevorzugt Binder aus den Gruppen Metalloxid-Sole, Cellulosederivate oder Alkalimetall-Silicate wie beispielsweise Siliziumoxid-Sole, Methylcellulosen oder Wasserglas eingesetzt werden.

10. Synthesereaktor nach einem der Ansprüche 1 bis 9, **dadurch gekennzeichnet, dass** die Reaktionsräume aus einem Baslsmaterial bestehen, das mindestens teilweise aus einem Edelstahl besteht.

11. Verfahren unter Einsatz des Synthesereaktors nach einem der Ansprüche 1 bis 10, **gekennzeichnet dadurch, dass**
der Reaktor isotherm betrieben wird und der maximale Temperaturanstieg zwischen Eintritt und Austritt des Synthesereaktors 5 K, vorzugsweise 2 K beträgt, wobei der Temperaturanstieg gleichermaßen für alle Bereiche innerhalb des Synthesereaktors gilt.

12. Verfahren nach Anspruch 11, **dadurch gekennzeichnet, dass**
in den Reaktionsräumen eine Temperatur von 100 bis 250 °C und vorzugsweise von 150 bis 200 °C vorliegt, wobei der Druck 1 bis 12 bar und vorzugsweise 6 bis 10 bar beträgt.

13. Verfahren nach einem der Ansprüche 11 bis 12, **dadurch gekennzeichnet, dass**
es bei explosiven Prozesszuständen durchgeführt wird und insbesondere der Sauerstoffgehalt im Prozessgas über 7 Vol.-% liegt.

## Claims

1. Synthesis reactor for producing vinyl acetate in which gaseous ethylene and acetic acid and also oxygen or oxygenous gases undergo catalytic reaction,
- the synthesis reactor is a wall reactor and
- comprises a multiplicity of reaction spaces in which the catalytic reaction is effected, wherein
- the free flow cross section in each of these reaction spaces is less than 2000 µm at least in one dimension, wherein
- at least one wall of the reaction spaces is coated with a catalyst, and
- at least one wall of the reaction spaces is indirectly cooled,
- the catalyst comprising from 0.3 to 10 wt% of palladium, from 0.2 to 5 wt% of gold and from 0.4 to 6 wt% of an alkali metal compound on an oxidic support material is adheringly applied to the wall of the reaction spaces with a binder.

2. Synthesis reactor according to Claim 1, **characterized in that** precisely one dimension of the reaction spaces is less than 1000 µm.

3. Synthesis reactor according to either of Claims 1 and 2, **characterized in that** the reaction spaces are formed from a multiplicity of tubes, or slots arranged from plates, wherein said tubes or slots may be arranged in any desired orientation and ideally run parallel to one another.

4. Synthesis reactor according to any of Claims 1 to 3, **characterized in that** the palladium content of the catalyst is from 0.8 to 5 wt%.

5. Synthesis reactor according to any of Claims 1 to 4, **characterized in that** the gold content of the catalyst is from 0.4 to 2.5 wt%.

6. Synthesis reactor according to any of Claims 1 to 5, **characterized in that** the alkali metal compound has an alkali metal content of from 1 to 4 wt%.

7. Synthesis reactor according to any of claims 1 to 6, **characterized in that** the catalyst comprises one or more elements from the group of alkaline earth metals, lanthanides, vanadium, iron, manganese, cobalt, nickel, copper, cerium, platinum, wherein the total proportion of these elements does not exceed 3 wt%.

8. Synthesis reactor according to any of Claims 1 to 7, **characterized in that** the oxidic support material is a metal oxide from the group SiO₂, Al₂O₃, TiO₂ and ZrO₂ as principal component, wherein in an advantageous embodiment the support material comprises further oxides as secondary components and the support material may be a natural mixed oxide from the group of bentonites.

9. Synthesis reactor according to any of Claims 1 to 8, **characterized in that** the binder is an oxidic or an organic binder, wherein it is preferable to use binders from the groups metal oxide sols, cellulose derivatives or alkali metal silicates, for example silicon oxide sols, methylcelluloses or waterglass.

10. Synthesis reactor according to any of Claims 1 to 9, **characterized in that** the reaction spaces are made of a base material which at least to an extent is made of stainless steel.

11. Process using the synthesis reactor according to any of Claims 1 to 10, **characterized in that** the reactor is operated isothermally and the maximum temperature increase between entry and exit of the synthesis reactor is 5 K, preferably 2 K, wherein the temperature increase applies equally for all areas inside the synthesis reactor.

12. Process according to Claim 11, **characterized in that** the temperature in the reaction spaces is from 100°C to 250°C and preferably from 150°C to 200°C, wherein the pressure is from 1 to 12 bar and preferably from 6 to 10 bar.

13. Process according to either of Claims 11 and 12, **characterized in that** it is carried out under explosive process conditions and, in particular, the oxygen content in the process gas is higher than 7 vol%.

## Revendications

1. Réacteur de synthèse pour la fabrication d'acétate de vinyle, dans lequel de l'éthylène gazeux et de l'acide acétique, ainsi que de l'oxygène ou des gaz contenant de l'oxygène réagissent catalytiquement,
- le réacteur de synthèse étant un réacteur à parois, et
- comprenant une pluralité de chambres de réaction, dans lesquelles la réaction catalytique a lieu,
- la section d'écoulement libre dans chacune de ces chambres de réaction étant inférieure à 2 000 µm dans au moins une dimension,
- au moins une paroi des chambres de réaction étant revêtue avec un catalyseur et
- au moins une paroi des chambres de réaction étant refroidie indirectement,
- le catalyseur, qui contient 0,3 à 10 % en poids de palladium, 0,2 à 5 % en poids d'or et 0,4 à 6 % en poids d'un composé de métal alcalin sur un matériau support oxydique, étant appliqué par adhésion au moyen d'un liant sur la paroi des chambres de réaction.

2. Réacteur de synthèse selon la revendication 1, **caractérisé en ce qu'**exactement une dimension des chambres de réaction est inférieure à 1 000 µm.

3. Réacteur de synthèse selon l'une quelconque des revendications 1 ou 2, **caractérisé en ce que** les chambres de réaction sont formées à partir d'une pluralité de tubes ou de fentes agencées à partir de plaques, ceux-ci pouvant être agencés dans une direction quelconque et étant idéalement parallèles les uns aux autres.

4. Réacteur de synthèse selon l'une quelconque des revendications 1 à 3, **caractérisé en ce que** la teneur en palladium du catalyseur est de 0,8 à 5 % en poids.

5. Réacteur de synthèse selon l'une quelconque des revendications 1 à 4, **caractérisé en ce que** la teneur en or du catalyseur est de 0,4 à 2,5 % en poids.

6. Réacteur de synthèse selon l'une quelconque des revendications 1 à 5, **caractérisé en ce que** le composé de métal alcalin présente une teneur en métal alcalin de 1 à 4 % en poids.

7. Réacteur de synthèse selon l'une quelconque des revendications 1 à 6, **caractérisé en ce que** le catalyseur contient un ou plusieurs éléments du groupe constitué par les métaux alcalino-terreux, les lanthanides, le vanadium, le fer, le manganèse, le cobalt, le nickel, le cuivre, le cérium, le platine, la proportion totale de ces éléments ne dépassant pas 3 % en poids.

8. Réacteur de synthèse selon l'une quelconque des revendications 1 à 7, **caractérisé en ce que** le matériau support oxydique est un oxyde métallique du groupe constitué par SiO₂, Al₂O₃, TiO₂ et ZrO₂ en tant que composant principal, le matériau support contenant selon une configuration avantageuse d'autres oxydes en tant que composants secondaires, et le matériau support pouvant être un oxyde mixte naturel du groupe des bentonites.

9. Réacteur de synthèse selon l'une quelconque des revendications 1 à 8, **caractérisé en ce que** le liant est un liant oxydique ou organique, des liants des groupes des sols d'oxydes de métaux, des dérivés de cellulose ou des silicates de métaux alcalins, tels que par exemple les sols d'oxyde de silicium, les méthylcelluloses ou le verre soluble, étant utilisés de préférence.

10. Réacteur de synthèse selon l'une quelconque des revendications 1 à 9, **caractérisé en ce que** les chambres de réaction sont constituées d'un matériau de base qui est constitué au moins partiellement d'un acier inoxydable.

11. Procédé utilisant le réacteur de synthèse selon l'une quelconque des revendications 1 à 10, **caractérisé en ce que** le réacteur est exploité de manière isotherme et l'élévation de température maximale entre l'entrée et la sortie du réacteur de synthèse est de 5 K, de préférence de 2 K, l'élévation de température étant uniforme pour toutes les zones dans le réacteur de synthèse.

12. Procédé selon la revendication 11, **caractérisé en ce qu'**une température de 100 à 250 °C et de préférence de 150 à 200 °C règne dans les chambres de réaction, la pression étant de 1 à 12 bar et de préférence de 6 à 10 bar.

13. Procédé selon l'une quelconque des revendications 11 à 12, **caractérisé en ce qu'**il est réalisé à des états de procédé explosifs, la teneur en oxygène du gaz de procédé étant notamment supérieure à 7 % en volume.
